(19) Europäisches Patentamt
European Patent Office
Office européen des brevets

(11) Veröffentlichungsnummer : **0 081 675 B1**

(12) **EUROPÄISCHE PATENTSCHRIFT**

(45) Veröffentlichungstag der Patentschrift : **30.12.86**

(51) Int. Cl.4 : **C 07 D 249/08**

(21) Anmeldenummer : **82110265.4**

(22) Anmeldetag : **08.11.82**

# (54) Alkylcycloalkyl-triazolylmethyl-ketone und Verfahren zu ihrer Herstellung.

(30) Priorität : **19.11.81 DE 3145857**

(43) Veröffentlichungstag der Anmeldung : **22.06.83 Patentblatt 83/25**

(45) Bekanntmachung des Hinweises auf die Patenterteilung : **30.12.86 Patentblatt 86/52**

(84) Benannte Vertragsstaaten : **AT BE CH DE FR GB IT LI NL**

(56) Entgegenhaltungen :
**EP-A- 0 080 102**
**DE-A- 2 201 063**
**DE-A- 2 905 981**
**DE-A- 3 020 500**
**Patent Abstracts of Japan, Band 5, Nr. 102, 2. Juli 1982**

(73) Patentinhaber : **BAYER AG**
**Konzernverwaltung RP Patentabteilung**
**D-5090 Leverkusen 1 Bayerwerk (DE)**

(72) Erfinder : **Krämer, Wolfgang, Dr.**
**Am Eckbusch 39/45**
**D-5600 Wuppertal 1 (DE)**
Erfinder : **Jautelat, Manfred, Dr.**
**Müllersbaum 28**
**D-5093 Burscheid (DE)**
Erfinder : **Kranz, Eckart, Dr.**
**Am Acker 9**
**D-5600 Wuppertal 1 (DE)**

**EP 0 081 675 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Alkylcycloalkyl-triazolylmethyl-ketone, ein Verfahren zu deren Herstellung und ihre Verwendung als Zwischenprodukte zur Synthese von Cycloalkyl-(α-triazolyl-β-hydroxy)-ketonen, welche fungizide und pflanzenwuchsregulierende Eigenschaften besitzen.

Es ist bereits bekannt geworden, daß bestimmte Alkyl- bzw. Phenyl-(α-triazolyl-β-hydroxy)-ketone gute fungizide Eigenschaften aufweisen (vgl. DE-OS 2 832 233). So lassen sich zum Beispiel 1,1,1-Trichlor-2-hydroxy-3-(1,2,4-triazol-1-yl)-4-(4-chlorphenyl)-butan-4-on und 2-Chlor-3-hydroxy-2,7,7-trimethyl-4-(1,2,4-triazol-1-yl)-heptan-5-on zur Bekämpfung von Pilzen einsetzen. Die Wirkung dieser Stoffe ist jedoch, insbesondere bei niedrigen Aufwandmengen und -konzentrationen, nicht immer ganz befriedigend.

Es wurden nun neue Alkylcycloalkyl-triazolylmethylketone der Formel

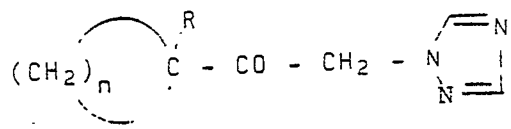
(I)

in welcher

R für geradkettiges oder verzweigtes Alkyl mit 1 bis 6 Kohlenstoffatome steht und

n für die Zahlen 3, 4, 5, 6 und 7 steht,

gefunden.

Weiterhin wurde gefunden, daß man die neuen Alkylcycloalkyl-triazolylmethyl-ketone der Formel (I) erhält, indem man Alkylcycloalkyl-halogenmethyl-ketone der Formel

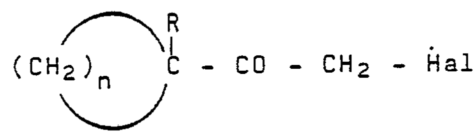
(II)

in welcher

R und n die oben angegebene Bedeutung haben und

Hal für Chlor oder Brom steht,

mit Triazol der Formel

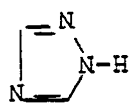
(III)

in Gegenwart eines Verdünnungsmittels und in Gegenwart eines Säurebindemittels umsetzt.

Die neuen Alkylcycloalkyl-triazolylmethyl-ketone sind interessante Zwischenprodukte zur Herstellung von Pflanzenschutz-Wirkstoffen. So eignen sich die Stoffe der Formel (I) als Ausgangsprodukte zur Synthese von Cycloalkyl-(α-triazolyl-β-hydroxy)-ketonen, welche sehr gute fungizide und pflanzenwuchsregulierende Wirksamkeit besitzen.

Überraschenderweise sind die Cycloalkyl-(α-triazolyl-β-hydroxy)-ketone, die sich aus den erfindungsgemäßen Alkylcycloalkyl-triazolylmethyl-ketonen der Formel (I) durch Umsetzung mit Aldehyden herstellen lassen, den aus dem Stand der Technik bekannten Verbindungen 1,1,1-Trichlor-2-hydroxy-3-(1,2,4-triazol-1-yl)-4-(4-chlorphenyl)-butan-4-on und 2-Chlor-3-hydroxy-2,7,7-trimethyl-4-(1,2,4-triazol-1-yl)-heptan-5-on bezüglich ihrer fungiziden Wirksamkeit überlegen. Darüber hinaus zeichnen sich die aus den erfindungsgemäßen Stoffe der Formel (I) herstellbaren Cycloalkyl-(α-triazolyl-β-hydroxy)-ketone unerwarteterweise auch durch sehr gute pflanzenwuchsregulierende Eigenschaften aus.

Die erfindungsgemäßen Stoffe sind durch die Formel (I) allgemein definiert.

Bevorzugt sind diejenigen Verbindungen der Formel (I), in denen R für Methyl oder Ethyl steht.

Im einzelnen seien außer den bei den Herstellungsbeispielen genannten Verbindungen folgende Verbindungen der Formel (I) genannt :

Tabelle 1

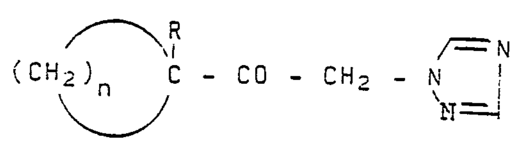
(I)

| R | n |
|---|---|
| $CH_3$ | 3 |
| $CH_3$ | 4 |
| $CH_3$ | 6 |
| $CH_3$ | 7 |

| R | n |
|---|---|
| $C_2H_5$ | 3 |
| $C_2H_5$ | 5 |
| $C_2H_5$ | 6 |
| $C_2H_5$ | 7 |

Verwendet man beispielsweise 1-Chloracetyl-1-ethylcyclopentan und 1,2,4-Triazol als Ausgangsstoffe, so kann der Reaktionsablauf bei dem erfindungsgemäßen Verfahren durch das folgende Formelschema wiedergegeben werden :

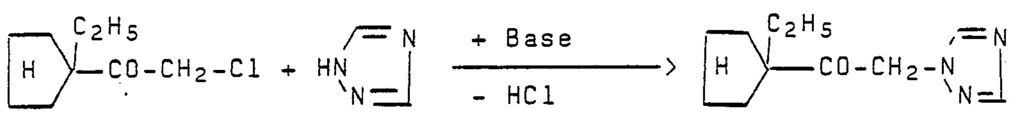


Die für die Durchführung des erfindungsgemäßen Verfahrens als Ausgangsstoffe benötigten Alkylcycloalkylhalogenmethylketone sind durch die Formel (II) allgemein definiert. In dieser Formel stehen R und der Index n vorzugsweise für diejenigen Bedeutungen, die bereits im Zusammenhang mit der Beschreibung der erfindungsgemäßen Stoffe der Formel (I) vorzugsweise für den Rest R bzw. den Index n aufgeführt wurden.

Die Alkylcycloalkyl-halogenmethyl-ketone der Formel (II) sind bisher noch nicht bekannt. Sie lassen sich jedoch nach im Prinzip bekannten Verfahren in einfacher Weise herstellen. So erhält man die Alkylcycloalkylhalogenmethyl-ketone der Formel (II), indem man 1,1-Dichloralkene der Formel

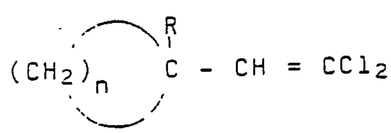
(IV)

in welcher R und n die oben angegebene Bedeutung haben,
mit Phenolaten der Formel

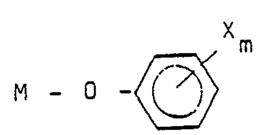
(V)

in welcher

M für ein Äquivalent eines Alkali- oder Erdalkalimetallions, insbesondere eines Natrium- oder Kaliumions steht,

X für Halogen, Alkyl oder Alkoxy mit jeweils 1 bis 3 Kohlenstoffatomen oder Phenyl steht und

m für 0, 1 oder 2 steht,

in Gegenwart eines inerten organischen Lösungsmittels, wie z. B. Dimethylformamid, bei Temperaturen zwischen 100 und 220 °C, gegebenenfalls unter erhöhtem Druck, umsetzt und die dabei erhaltenen

Phenylether der Formel

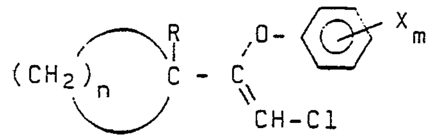

(VI)

in welcher R, X, m und n die oben angegebene Bedeutung haben in üblicher Weise mit Mineralsäuren, wie z. B. Schwefelsäure oder Salzsäure, und/oder mit organischen Säuren, wie z. B. Ameisensäure, bei 40 bis 100 °C hydrolysiert.

Die Herstellung von 1,1-Dichloralkenen der Formel (IV) ist bekannt. Sie erfolgt durch Addition von Alkylhalogeniden an Vinylidenchlorid in Gegenwart von sauren Katalysatoren (vgl. hierzu J. Am. Chem. Soc. *74,* 2885 (1952)) mit gleichzeitiger Abspaltung von Halogenwasserstoff.

Die Phenolate der Formel (V) sind allgemein bekannte Verbindungen der organischen Chemie.

Die Alkylcycloalkyl-halogenmethyl-ketone der Formel (II) können auch erhalten werden, indem man Alkylcycloalkyl-methyl-ketone der Formel

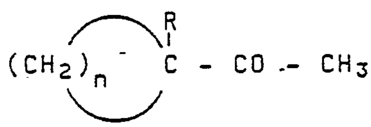

(VII)

in welcher R und n die oben angegebene Bedeutung haben, in üblicher Weise mit Chlor oder Brom in Gegenwart eines inerten organischen Lösungsmittels, wie beispielsweise Ether, chlorierten oder nichtchlorierten Kohlenwasserstoffen, bei Raumtemperatur umsetzt, oder mit üblichen Chlorierungsmitteln, wie beispielsweise Sulfurylchlorid, bei 20 bis 60 °C umsetzt.

Die Alkylcycloalkyl-methyl-ketone der Formel (VII) werden erhalten, indem man entsprechende Nitrile der Formel

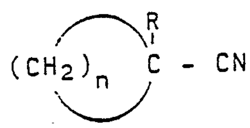

(VIII)

in welcher R und n die oben angegebene Bedeutung haben, in üblicher Weise mit metallorganischen Verbindungen, wie insbesondere Methylmagnesiumbromid, in Gegenwart eines Verdünnungsmittels, wie z. B. eines wasserfreien Ethers, bei Temperaturen zwischen 0 und 80 °C umsetzt.

Die Nitrile der Formel (VIII) sind bekannt (vgl. Journal of Organometallic Chemistry *57,* C 33-35 (1973)) bzw. können sie nach dem dort angegebenen Verfahren erhalten werden.

Für das erfindungsgemäße Verfahren kommen als Verdünnungsmittel inerte organische Lösungsmittel infrage. Hierzu gehören vorzugsweise Ketone, wie Aceton, Methylethylketon und Methylbutylketon ; Alkohole, wie Ethanol, Isopropanol und Butanol ; aromatische Kohlenwasserstoffe, wie Benzol und Toluol ; Formamide und Sulfoxide, wie Dimethylformamid und Dimethylsulfoxid.

Das erfindungsgemäße Verfahren wird in Gegenwart eines Säurebindemittels vorgenommen. Man kann alle üblicherweise verwendbaren anorganischen oder organischen Säurebinder zugeben, wie Alkalicarbonate, beispielsweise Natriumcarbonat, Kaliumcarbonat und Natriumhydrogencarbonat, wie Alkali- und Erdalkalihydroxide, beispielsweise Kaliumhydroxid und Calciumhydroxid, oder wie niedere tertiäre Alkylamine, Cycloalkylamine oder Aralkylamine, beispielsweise Triethylamin, N,N-Dimethylmethylcyclohexylamin, Dicyclohexylamin, N,N-Dimethylbenzylamin, weiterhin Pyridin und Diazabicyclooctan.

Vorzugsweise verwendet man einen entsprechenden Überschuß an Triazol.

Die Reaktionstemperaturen können bei dem erfindungsgemäßen Verfahren in einem größeren Bereich variiert werden. Im allgemeinen arbeitet man bei Temperaturen zwischen 0 und 120 °C, vorzugsweise zwischen 20 und 90 °C. Zweckmäßigerweise arbeitet man beim Siedepunkt des jeweiligen Lösungsmittels.

Bei der Durchführung des erfindungsgemäßen Verfahrens setzt man auf 1 Mol der Verbindungen der Formel (II) vorzugsweise 1 bis 4 Mol Azol und 1 bis 4 Mol Säurebinder ein. Zur Isolierung der Verbindungen der Formel (I) wird das Lösungsmittel abdestilliert und der Rückstand in üblicher Art und Weise aufgearbeitet.

Die erfindungsgemäßen Alkylcycloalkyl-triazolylmethylketone der Formel (I) eignen sich als Zwischenprodukte zur Synthese von Cycloalkyl-(α-triazolyl-β-hydroxy)-ketonen, welche fungizide und

pflanzenwuchsregulierende Wirksamkeit besitzen (vgl. EP-A-0 080 102).

Solche Cycloalkyl-(α-triazolyl-β-hydroxy)-ketone der Formel

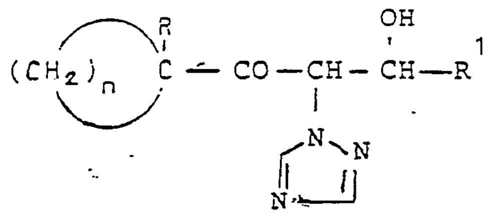

(IX)

in welcher

R und n die oben angegebene Bedeutung haben und

$R^1$ für Halogenalkyl, Halogenalkenyl oder Alkoxycarbonyl steht, lassen sich herstellen, indem man Alkylcycloalkyl-triazolyl-methyl-ketone der Formel

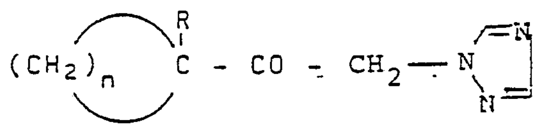

(I)

in welcher R und n die oben angegebene Bedeutung haben, mit Aldehyden der Formel

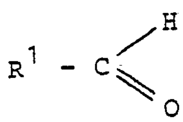

(X)

in welcher $R^1$ die oben angegebene Bedeutung hat,

in Gegenwart eines Verdünnungsmittels und in Gegenwart eines Katalysators umsetzt.

Die bei dieser Umsetzung als Reaktionskomponenten zu verwendenden Aldehyde sind durch die Formel (X) allgemein definiert. In dieser Formel steht $R^1$ vorzugsweise für geradkettiges oder verzweigtes Halogenalkyl mit 1 bis 4 Kohlenstoffatomen und 1 bis 5 gleichen oder verschiedenen Halogenatomen, wie insbesondere Fluor, Chlor oder Brom, geradkettiges oder verzweigtes Halogenalkenyl mit 2 bis 4 Kohlenstoffatomen und 1 bis 5 gleichen oder verschiedenen Halogenatomen, wie insbesondere Fluor, Chlor oder Brom, sowie für Alkoxycarbonyl mit 1 bis 4 Kohlenstoffatomen im Alkoxyteil.

Die Aldehyde der Formel (X) sind allgemein bekannte Verbindungen der organischen Chemie.

Für die Umsetzung der erfindungsgemäßen Alkylcycloalkyltriazolylmethyl-ketone der Formel (I) mit Aldehyden der Formel (X) kommen als Verdünnungsmittel alle inerten organischen Solventien in Frage. Vorzugsweise in Betracht kommen Alkohole, wie Methanol und Ethanol, sowie deren Mischungen mit Wasser ; Ether, wie Tetrahydrofuran und Dioxan ; Nitrile, wie Acetonitril und Propionitril ; halogenierte aliphatische und aromatische Kohlenwasserstoffe, wie Methylenchlorid, Tetrachlorkohlenstoff, Chloroform, Chlorbenzol und Dichlorbenzol ; sowie Eisessig.

Das oben angegebene Verfahren zur Herstellung von Cycloalkyl-(α-triazolyl-β-hydroxy)-ketonen wird in Gegenwart eines Katalysators durchgeführt. Dabei kann man alle üblicherweise für derartige Reaktionen verwendbaren sauren und insbesondere basischen Katalysatoren einsetzen. Hierzu gehören vorzugsweise Lewis-Säuren, wie z. B. Eisen-(III)-chlorid, Eisen-(III)-bromid, Bortrifluorid, Bortrichlorid, Zinntetrachlorid oder Titantetrachlorid ; ferner Alkali- und Erdalkalihydroxide, wie Kaliumhydroxid, Natriumhydroxid, Calciumhydroxid oder Bariumhydroxid ; außerdem Alkalisalze, wie Kaliumcarbonat, Natriumcarbonat, Kaliumcyanid, sekundäres Natriumphosphat, Natriumacetat und Natriumsulfit ; sowie Alkoholate, wie Natrium- oder Kaliumethylat.

Die Reaktionstemperaturen können bei dem oben angegebenen Verfahren zur Herstellung von Cycloalkyl-(α-triazolyl-β-hydroxy)-ketonen in einem größeren Bereich variiert werden. Im allgemeinen arbeitet man bei Temperaturen zwischen 0 °C und 100 °C, vorzugsweise bei Raumtemperatur bzw. dem Siedepunkt des jeweiligen Lösungsmittels.

Bei der Durchführung des oben angegebenen Verfahrens zur Herstellung von Cycloalkyl-(α-triazolyl-β-hydroxy)-ketonen setzt man die Reaktionskomponenten der Formeln (I) und (X) im allgemeinen in äquimolaren Mengen ein. Außerdem fügt man eine katalytische oder auch äquimolare Mengen an Katalysator hinzu. Es ist auch möglich, eine der Reaktionskomponenten der Formeln (I) und (X) in einem Überschuß einzusetzen. Die Isolierung der Verbindungen der Formel (IX) erfolgt in üblicher Weise.

Die aus den erfindungsgemäßen Stoffen herstellbaren Cycloalkyl-(α-triazolyl-β-hydroxy)-ketone der Formel (IX) besitzen sehr gute fungizide und pflanzenwuchsregulierende Eigenschaften. Insbesondere

können sie zur Bekämpfung solcher Pilze eingesetzt werden, die echte Mehltauerkrankungen hervorrufen.

## Herstellungsbeispiele

### Beispiel 1

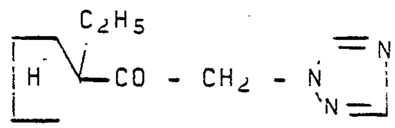

52,5 g (0,3 Mol) 1-Chlor-acetyl-ethylcyclopentan, 42 g Kaliumcarbonat und 72 g 1,2,4-Triazol werden in 400 ml Ethanol gelöst und 48 Stunden bei 20 °C gerührt. Das Lösungsmittel wird im Vakuum abdestilliert, der Rückstand in 500 ml Wasser aufgenommen und mit 300 ml Methylenchlorid verrührt. Man trennt die organische Phase ab, wäscht mehrmals mit je 100 ml Wasser, trocknet über Natriumsulfat und engt im Vakuum ein. Der Rückstand kristallisiert langsam durch. Man erhält 50 g (81 % der Theorie) 1-Ethylcyclopentyl-(1,2,4-triazol-1-yl)-methylketon mit dem Brechungsindex $n_D^{20}$ = 1,502 7.

Herstellung des Ausgangsproduktes

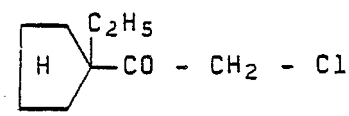

(II-1)

125,3 g (0,5 Mol) 1-Chlor-2-(1-ethylcyclopentyl)-2-phenoxyethylen werden in 500 ml Ameisensäure und 50 ml konzentrierter Salzsäure 2 Stunden auf 80 °C erwärmt.

Dann wird mit Methylenchlorid und Eis verdünnt und dreimal mit 2n Natronlauge ausgeschüttelt. Nach dem Trocknen der Methylenchloridphase über Natriumsulfat wird das Lösungsmittel im Vakuum abrotiert. Der Rückstand wird im Vakuum destilliert. Man erhält 72 g (82,6 % der Theorie) 1-Chloracetyl-1-ethylcyclopentan vom Brechungsindex $n_D^{20}$ = 1,484.

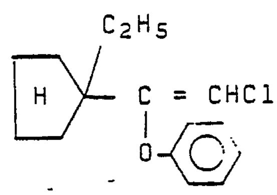

(VI-1)

140 g (4,2 Mol) Natriumphenolat werden in 500 ml N-Methylpyrrolidon auf 200 °C erhitzt. 116 g (0,6 Mol) 1-(2,2-Dichlorvinyl)-1-ethyl-cyclopentan werden so langsam zugetropft, daß die Reaktionstemperatur nicht unter 195 °C sinkt. Dann wird 1 Stunde bei 210 °C nachgerührt. Nach dem Abkühlen wird mit Methylenchlorid verdünnt und mit 2n Natronlauge mehrfach ausgeschüttelt. Die über Natriumsulfat getrocknete organische Phase wird im Vakuum eingeengt, und der Rückstand wird fraktioniert. Man erhählt 132 g (88 % der Theorie) 1-Chlor-2-(1-ethylcyclopentyl)-2-phenoxy-ethylen vom Siedepunkt 115-125 °C/0,1 Torr.

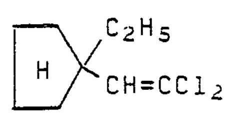

(IV-1)

Zu 291 g (3 Mol) 1,1-Dichlorethen werden bei — 20 °C 10 g wasserfreies Aluminiumchlorid gegeben und danach bei 0-10 °C 133 g (1 Mol) 1-Ethyl-cyclopentylchlorid (Chem. Abstr. *42*, 6328 (1948)) eingetropft. Man läßt die Reaktionslösung auf 20 °C erwärmen, setzt weitere 5 g Aluminiumchlorid zu und rührt 2 Stunden bei 20 °C nach. Der Ansatz wird auf Eis gegossen, mit Methylenchlorid und verdünnter Salzsäure aufgearbeitet. Aus der organischen Phase gewinnt man dach Trocknen über Natriumsulfat durch franktionierte Destillation 158 g (82 % der Theorie) 1-(2,2-Dichlorvinyl)-1-ethyl-

cyclopentan vom Siedepunkt 45-50 °C/0,1 Torr.

In analoger Weise und entsprechend dem erfindungsgemäßen Verfahren werden die in der Tabelle 2 aufgeführten Verbindungen der Formel

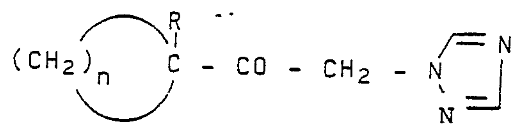

(I)

erhalten :

Tabelle 2

| Beispiel Nr. | n | R | Schmelzpunkt (°C) bzw. Brechungsindex $n_D^{20}$ |
|---|---|---|---|
| 2 | 5 | $CH_3$ | 1,5058 |

Entsprechend Beispiel 1 und gemäß den angegebenen Verfahren werden die in der Tabelle 3 aufgeführten Ausgangsstoffe der Formel

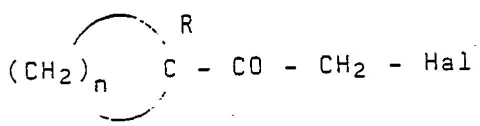

(II)

erhalten :

Tabelle 3

| Beispiel Nr. | n | R | Hal | Physikal. Konstante Kp °C (mbar) |
|---|---|---|---|---|
| (II-2) | 5 | $CH_3$ | Cl | $Kp_{20}$ 115 |
| (II-3) | 4 | $C_4H_9$ | Cl | $Kp_{0,1}$ 82-88 |
| (II-4) | 6 | $CH_3$ | Cl | $Kp_{0,1}$ 75-78 |
| (II-5) | 7 | $CH_3$ | Cl | $Kp_{0,3}$ 92-96 |

Herstellung eines Folgeproduktes

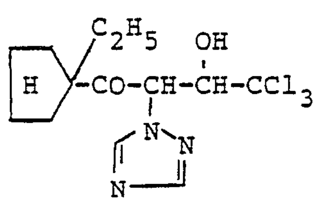

(IX-1)

Zu 10,4 g (0,05 Mol) 1-Ethylcyclopentyl-(1,2,4-triazol-1-yl)-methylketon (Beispiel 1) und 13,8 g (0,1 Mol) gemahlenem Kaliumcarbonat in 100 ml Tetrahydrofuran werden 9,3 g (0,064 Mol) Chloral getropft. Man läßt das Reaktionsgemisch über Nacht bei Raumtemperatur rühren und verrührt es anschließend mit Wasser. Der entstandene Feststoff wird abgesaugt und aus Essigester umkristallisiert. Man erhält 7 g (39,5 % der Theorie) 1,1,1-Trichlor-2-hydroxy-3-(1,2,4-triazol-1-yl)-4-(1-ethylcyclopentan-1-yl)-butan-4-on vom Schmelzpunkt 194-96 °C.

**Patentansprüche**

1. Alkylcycloalkyl-triazolylmethyl-ketone der Formel

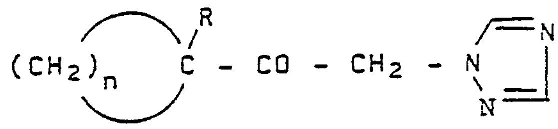

(I)

in welcher
R für geradkettiges oder verzweigtes Alkyl mit 1 bis 6 Kohlenstoffatomen steht und
n für die Zahlen 3, 4, 5, 6 und 7 steht.

2. Verfahren zur Herstellung von Alkylcycloalkyltriazolylmethyl-ketonen der Formel

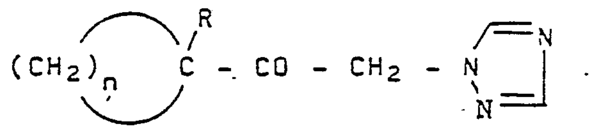

(I)

in welcher
R für geradkettiges oder verzweigtes Alkyl mit 1 bis 6 Kohlenstoffatomen steht und
n für die Zahlen 3, 4, 5, 6 und 7 steht,
dadurch gekennzeichnet, daß man Alkylcycloalkylhalogenmethyl-ketone der Formel

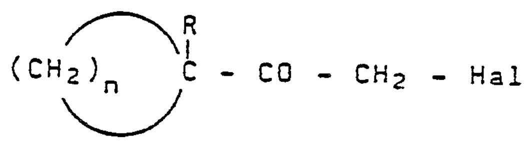

(II)

in welcher
R und n die oben angegebene Bedeutung haben und
Hal für Chlor oder Brom steht,
mit Triazol der Formel

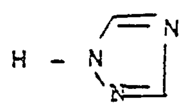

(III)

in Gegenwart eines Verdünnungsmittels und in Gegenwart eines Säurebindemittels umsetzt.

**Claims**

1. Alkylcycloalkyl triazolylmethyl ketones of the formula

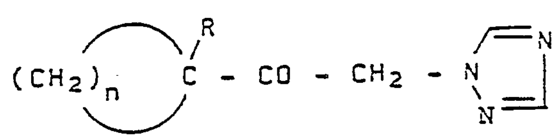

(I)

in which
R represents straight-chain or branched alkyl with 1 to 6 carbon atoms and
n represents the numbers 3, 4, 5, 6 and 7.

2. Process for the preparation of alkylcycloalkyl triazolylmethyl ketones of the formula

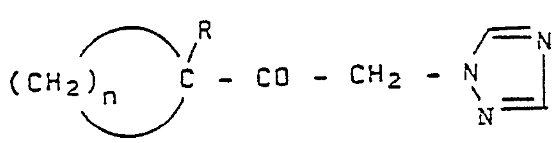

(I)

in which

R represents straight-chain or branched alkyl with 1 to 6 carbon atoms and

n represents the numbers 3, 4, 5, 6 and 7,

characterised in that alkylcycloalkyl halogenomethyl ketones of the formula

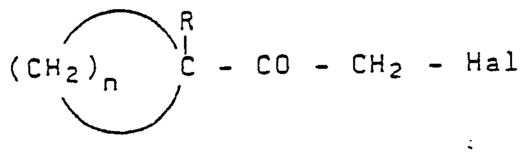

(II)

in which

R and n have the abovementioned meaning and

Hal represents chlorine or bromine,

are reacted with triazole of the formula

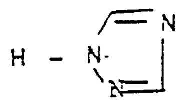

(III)

in the presence of a diluent and in the presence of an acid-binding agent.

**Revendications**

1. Alkylcycloalkyl-triazolylméthyl-cétones de formule

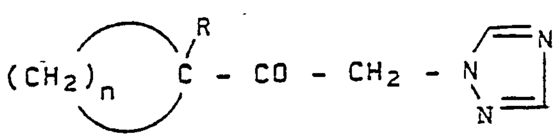

(I)

dans laquelle

R est un groupe alkyle à chaîne droite ou ramifié ayant 1 à 6 atomes de carbone et

n représente les nombres 3, 4, 5, 6 et 7.

2. Procédé de production d'alkylcycloalkyltriazolylméthyl-cétones de formule

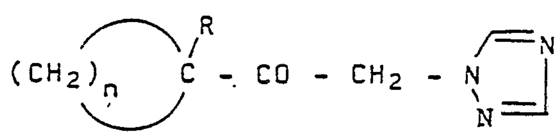

(I)

dans laquelle

R est un groupe alkyle à chaîne droite ou ramifié ayant 1 à 6 atomes de carbone et

n représente les nombres 3, 4, 5, 6 et 7,

caractérisé en ce qu'on fait réagir en présence d'un diluant et en présence d'un accepteur d'acide, des alkylcycloalkyl-halogénométhyl-cétones de formule

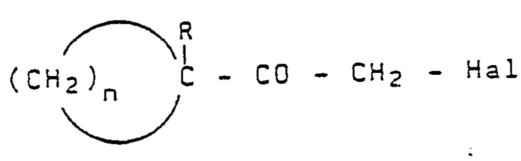

(II)

dans laquelle

R et n ont la définition indiquée ci-dessus et

Hal désigne le chlore ou le brome, avec le triazole de formule

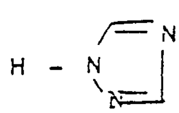

(III)